Europäisches Patentamt

European Patent Office

Office européen des brevets

(19)

(11) Publication number : **0 483 072 A1**

(12) **EUROPEAN PATENT APPLICATION**

(21) Application number : **91830039.3**

(22) Date of filing : **08.02.91**

(51) Int. Cl.⁵ : **A61N 1/22**

(30) Priority : **26.10.90 IT 492790 U**

(43) Date of publication of application :
**29.04.92 Bulletin 92/18**

(84) Designated Contracting States :
**AT BE CH DE DK ES FR GB GR LI LU NL SE**

(71) Applicant : **VUPIESSE ITALIA S.A.S., di VALENTINI E PAOLIZZI E C.**
**Via Destra del Porto, 113**
**I-47037 Rimini (Forli) (IT)**

(72) Inventor : **Valentini, Marco**
**Via Borsi, 4**
**I-47037 Rimini (Forli) (IT)**
Inventor : **Paolizzi, Marco**
**Via Lince, 18**
**I-47037 Rimini (Forli) (IT)**

(74) Representative : **Lanzoni, Luciano**
**c/o BUGNION S.p.A. Via Cairoli, 107**
**I-47037 Rimini (Forli) (IT)**

(54) **Passive exercise device producing a firming and slimming action with electrical impulses emitted by a generator and transmitted through electrodes placed in contact with the skin.**

(57) The exerciser firms and slims utilizing impulses emitted by a generator (4) and transmitted through electrodes (2) which are mounted slidably to a flexible belt (1) and positioned in contact with the skin of the user ; the generator is accommodated internally of a pocket (3) incorporated into the belt, and wired up to the electrodes (2) by way of leads (5) not less in length than a given maximum degree of infinitely variable adjustment allowed to the electrodes (2) in relation to the longitudinal dimension of the belt.

FIG.1

EP 0 483 072 A1

Jouve, 18, rue Saint-Denis, 75001 PARIS

The invention relates to a passive exerciser that firms and slims by means of impulses emitted from a generator and transmitted through electrodes placed in contact with the skin of the user.

Hitherto, such appliances have consisted in a sling or harness worn by the user and provided with slots into which a given number of electrodes, wired to a generator of electric current physically remote from the harness, would be inserted in such a way as to remain supported in contact with the skin at positions corresponding to those areas selected for stimulation by the electrical impulses.

In more streamlined embodiments of such exercisers, the sling or harness is entirely eliminated and the single electrodes held in place by strappings or sticking plasters attached locally to the skin. These former appliances were inconvenient in use and by no means free of more specific drawbacks, first among which being that the generator, remote from the harness, was connected to the electrodes by way of leads and thus cumbersome to carry about; a user would therefore be constrained to immobility while exercising in order to avoid any accidental disconnection of the leads from the generator.

To further disadvantage moreover, except in designs where use was made of sticking plaster to hold the electrodes in place, the provision of slots in the sling or harness meant that the electrodes could be located only in certain prescribed positions, not always coinciding with the exact areas selected for stimulation; and whilst a given degree of mobility afforded by the component parts of the harness may indeed have afforded a certain indirect freedom of adjustment to the electrodes, it is equally clear that any accurate positioning of the electrodes, if at all obtainable, was laborious and difficult to sustain throughout an entire session of use.

Evidently, such a drawback would not present itself in the case of devices using sticking plasters or other similar expedients, though the difficulties in this case are no less evident, especially where the electrodes are offered to unshaven skin.

In addition, all such conventional devices required the use of a conductive paste or gel in order to ensure an effective degree of contact between the electrodes and the skin (indispensable if efficient conduction is to be assured). Such substances would be spread over the areas selected for stimulation prior to positioning the electrodes, and though the skin is not soiled by their application, there is nonetheless a certain inconvenience caused to the user particularly as regard; the need to 'clean up' on termination of the session.

Accordingly, the object of the present invention is to overcome the drawbacks described above.

The stated object is fully realized in a passive exercise device as characterized in the appended claims; such a device, which produces a firming and slimming action using electrical impulses emitted by a generator of electric current and transmitted through electrodes placed in contact with the skin, comprises a longitudinal element fashioned in readily deformable material, slidably supporting the electrodes and worn by the user in such a way as to enable their direct contact with the skin. The generator is housed in a pocket afforded by the longitudinal element, and wired to the electrodes by way of leads not less in length than the maximum degree of adjustment allowed to the electrodes in relation to the longitudinal element; furthermore, the position of the electiodes in relation to the longitudinal element is infinitely variable. The fundamental advantage afforded by the present invention is thus reflected in the creation of a portable passive exercise device in which the position of the electrodes is infinitely variable, and which can be put on by the user even under normal clothing in such a manner, for instance, as to permit of exercising with the device while at the same time carrying on a wide variety of motor functions unhindered in any way.

A further advantage derives from the fact that the electrodes are covered by a pad of sponge located between the electrode itself and the skin, which when moistened with water ensures the necessary conductivity and spares the user the inconvenience of employing a gel.

Furthermore, besides being removable and, amongst other benefits, allowing a more hygienic use of the exerciser whether by one or by more individuals, the sponge pads also prevent the metal electrodes from entering into direct contact with the skin, thereby eliminating the unpleasant cold sensation experienced on initial contact with the electrodes of conventional exercisers as described above.

The invention will now be described in detail, by way of example, with the aid of the accompanying drawings, in which:

– fig 1 is an illustration of the exerciser in its entirety, viewed in perspective;

– fig 2 illustrates a detachable component of the exerciser of fig 1;

– fig 3 shows a detail of the invention, exploded in perspective and on larger scale better to reveal certain internal features;

– fig 4 is the section through IV-IV of fig 1. Referring to fig 1, a passive exercise device 50 according to the invention for firming and slimming the body will be seen to comprise a generator 4 of electric current, and a plurality of electrodes 2 wired to the generator and applied direct to the skin, by which electrical impulses having suitable characteristics are transmitted to the cutis.

The device 50 comprises a longitudinal element 1, embodied preferably as a belt designed to be worn by the user, and consisting in two strips 8a, 8b of readily deformable plastic material united one to the other and secured along respective peripheral edges 9 in such a way as will create a pocket 3 internally of which to accommodate the generator 4.

The pocket 3 is designed also to house the power supply for the generator 4 (this will consist in a conventional battery not shown in the drawings), and accessible from externally of the longitudinal element 1 by way of an opening 31 concealed beneath a flap 32 integral with the element itself.

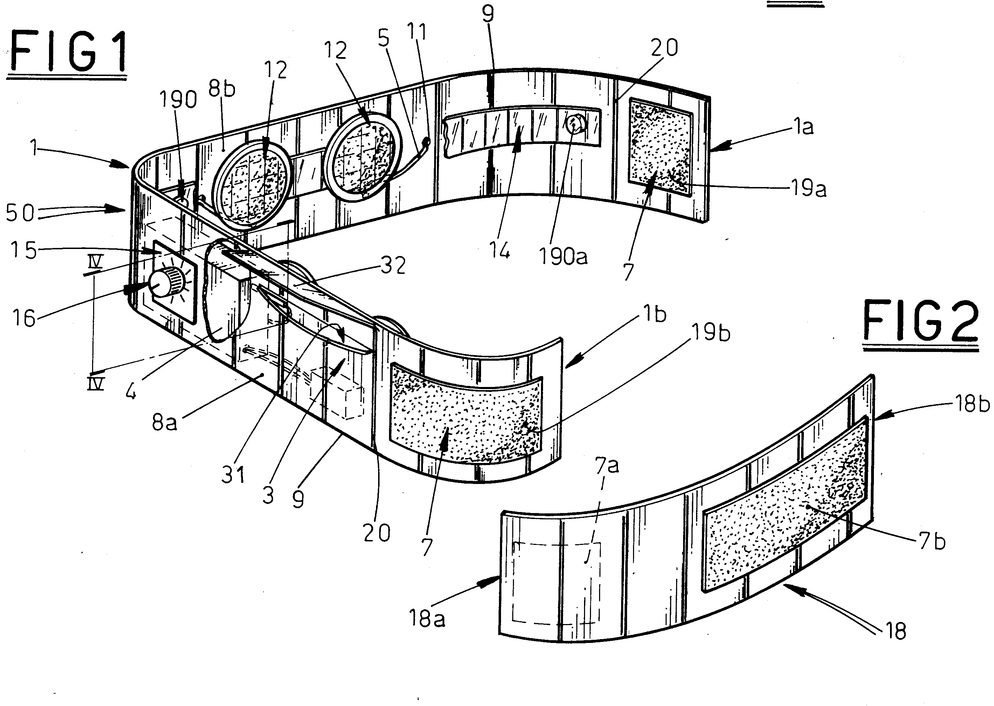

With reference in particular to fig 1, it will be seen that the strips 8a, 8b of the belt afford a plurality of surface grooves 20, e.g. consisting in transverse stitching, by which the deformability of the longitudinal element 1 is enhanced to the end of ensuring closer contact between the electrodes 2 and the skin.

7 denotes means by which to fasten the two opposite ends 1a and 1b of the longitudinal element 1 one to the other, embodied and positioned in such a way that the element 1 can fit around parts of the body that are dissimilar in proportion.

In a preferred embodiment, such fastening means 7 will consist in velcro patches 19a and 19b attached permanently to the respective ends 1a and 1b of the longitudinal element 1, which grip when overlapped and thus secure the belt in a loop.

The longitudinal element 1 will also incorporate a panel 15, associated with one of the two strips 8a or 8b, affording a knob 16 by means of which the generator 4 can be switched on and adjusted by the wearer of the exerciser.

Advantageously, the device 50 further comprises an extension element 18 (see fig 2), the two ends 18a and 18b of which afford relative velcro patches 7a and 7b that can be fastened temporarily to the corresponding ends 1a and 1b of the longitudinal element 1 in such a way as to increase the length of the belt, if need be.

The electrodes 2 are supported slidably by the longitudinal element 1, associated with a track 14 fastened in place by relative means 190 consisting preferably in screws or press studs 190a, such that the position of the electrodes 2 along the track 14 is rendered adjustable and infinitely variable in relation to the longitudinal element 1.

Being thus removable, such fastening means 190 allow the endmost electrodes 2 to be detached or reversed, and thus excluded, to the end of using two electrodes only for special applications.

The electrodes 2 are wired to the generator 4 by way of respective conductors 5 accommodated within and permanently concealed by the pocket 3, which emerge through holes 11 located in one of the two strips 8a or 8b. The conductors 5 will be of length not less than the maximum distance through which the single electrode 2 can be positioned along the track 14, in order to ensure that no restriction is placed on the full range of adjustment allowed to the electrodes 2 in relation to the longitudinal element 1.

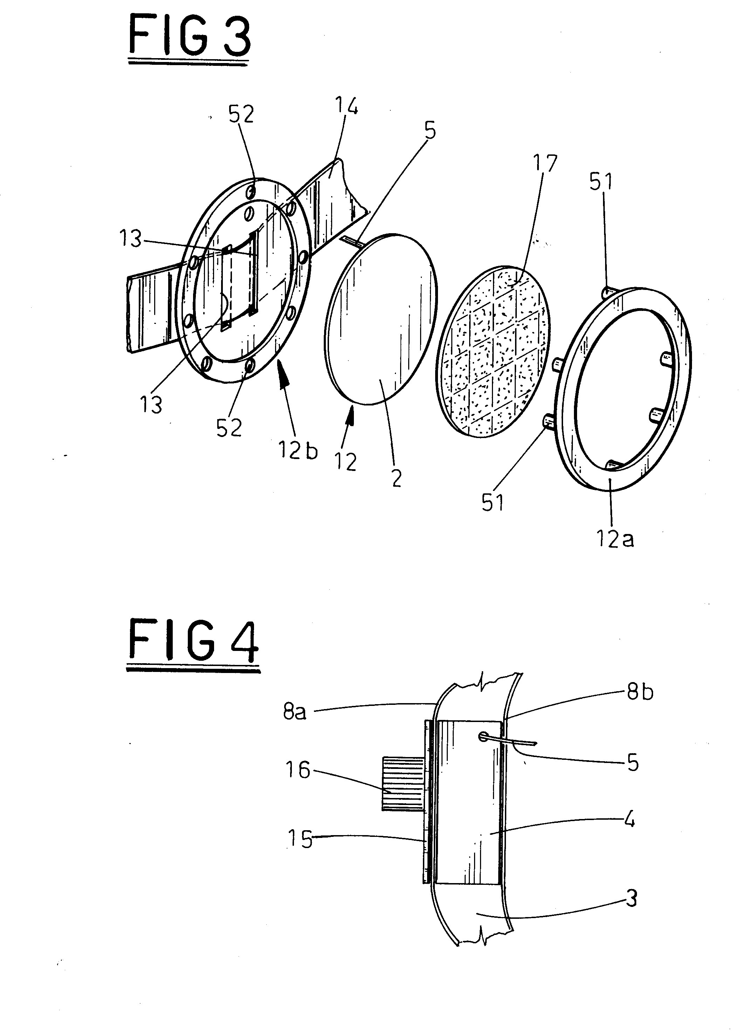

In a preferred embodiment of the device shown in fig 3, the single electrode 2 is carried internally of a support 12 affording a slot 13 which couples slidably with the track 14. The support 12 consists in two matching halves 12a and 12b, fashioned from a plastics material and designed to fasten together with the metal electrode 2 sandwiched between.

A first half 12a of the support 12 is embodied with pins 51 capable of frontal insertion and separation into and from corresponding sockets 52 afforded by the remaining half 12b, such that the two parts can be held stably together.

In addition, one half 12a of the support 12 affords a central opening through which the corresponding electrode 2 is offered to the skin of the wearer. As illustrated in the example of fig 3, each of the electrodes 2 is associated with a layer or pad 17 of material, preferably sponge, sandwiched together with the electrode between the two halves 12a and 12b of the support and offered to the skin, which will be moistened'prior to use with an electrically conductive liquid, preferably water, in order to ensure the passage of current from the electrode 2 to the cutis of the wearer and thus eliminate the inconvenience of applying and removing gels to and from the skin before and after using the device.

## Claims

1) A passive exercise device, designed to produce a firming and slimming action using electrical impulses emitted by a generator of electric current and transmitted through electrodes placed in contact with the skin,
characterized
in that it comprises at least one longitudinal element (1) worn by the user, slidably supporting a plurality of electrodes (2) and affording at least one pocket (3) in which to accommodate a generator of electric current (4) that is connected to the electrodes by way of conductors (5) equivalent in length at least to a maximum degree of adjustment allowed to the corresponding electrodes in relation to the longitudinal element; and in that the position of the electrodes in relation to the longitudinal element is infinitely variable.

2) A device as in claim 1, wherein the two opposite ends (1a, 1b) of the longitudinal element (1) are provided with fastening means (7) such as afford an infinitely variable adjustment of the position at which the ends (1a, 1b) are fastened.

3) A device as in claim 1, wherein the longitudinal element (1) is embodied as a belt to be worn by a user, comprising at least two strips (8a, 8b) of material matched one to the other and united along their respective peripheral edges in such a way as to encompass the pocket (3) internally of the longitudinal element and permit of accommodating and concealing the conductors (5) internally of the pocket (3), save at the connections with the corresponding electrodes (2) where the respective ends of the conductors emerge through holes (11) located in at least one of the two strips (8a, 8b).

4) A device as in claim 1, wherein the electrodes (2) are carried by respective supports (12) slidably associated with a track (14) connected to the longitudinal element (1), in such a way as to allow infinitely variable adjustment of the position of the electrodes (2) in relation to the longitudinal element (1).

5) A device as in claim 1, wherein the longitudinal element (1) comprises a panel (15) equipped with at least one knob (16) by means of which to operate and regulate the generator (4) of electric current.

6) A device as in claim 1, wherein the length of the longitudinal element can be extended by fastening the opposite ends (1a, 1b) temporarily to the corresponding opposite ends (7a, 7b) of a further longitudinal element (18).

7) A device as in claim 1, wherein the pocket (3) is accessible by way of an opening (13) concealed beneath a flap (32) embodied integrally with the longitudinal element (1).

8) A device as in claim 2, wherein fastening means (7) are embodied as patches (19a, 19b) of velcro type material permanently associated with the ends (1a, 1b) of the longitudinal element (1).

9) A device as in claim 3, wherein the strips (8a, 8b) of the longitudinal element (1) are fashioned from a readily deformable plastic material.

10) A device as in claim 4, wherein the supports (12) are embodied in at least two matching halves (12a, 12b) that can be fastened one to the other with the respective electrodes (2) sandwiched between, of which the half (12a) nearest the skin affords a central opening enabling the respective electrode to enter into contact with the skin of the wearer.

11) A device as in claim 4, wherein each support (12) comprises at least one slot (13) associated with at least one of the halves (12a, 12b), through which the track (14) is slidably insertable.

12) A device as in claim 4, wherein the track (14) is associated with the longitudinal element (1) by way of relative detachable fastening means (190).

13) A device as in claim 9, wherein the strips (8a, 8b) exhibit transverse grooves (20) serving to enhance the deformability of the longitudinal element (1).

14) A device as in claim 10, wherein the one half (12a) of the support (12) affords pins (51) insertable into and removable from corresponding sockets (52) afforded by the matching half (12b).

15) A device as in claim 10, wherein each electrode (2) is sandwiched between the two halves (12a, 12b) of the relative support (12) together with a layer (17) of sponge directed toward the skin of the user and moistened with electrically conductive liquid.

16) A device as in claim 12, wherein the detachable fastening means (190) consist in screws (190a).

17) A device as in claim 12, wherein the detachable fastening means (190) consist in press studs (190a).

18) A device as in claim 15, wherein the electrically conductive liquid is water.

FIG 1

FIG 2

# FIG 3

# FIG 4

EP 0 483 072 A1

European Patent Office

EUROPEAN SEARCH REPORT

Application Number

| | DOCUMENTS CONSIDERED TO BE RELEVANT | EP 91830039.3 | |
|---|---|---|---|

| Category | Citation of document with indication, where appropriate, of relevant passages | Relevant to claim | CLASSIFICATION OF THE APPLICATION (Int. Cl.5) |
|---|---|---|---|
| A | GB - A - 2 109 689 (MIDDLETON) * Fig. 1; description * | 1,2, 3,8, 9 | A 61 N 1/22 |
| A | EP - A1 - 0 242 546 (SIEMENS) * Description; fig. * | 1,4, 15 | |
| A | DE - A1 - 3 618 933 (MASAKI) * Abstract; fig. 1-4 * | 1,2, 3,9, 15 | |
| A | DE - A1 - 3 921 784 (EHLERS) * Claims 1,2 * | 1,2, 8,9 | |
| A | DD - A - 206 070 (SCHWESINGER) * Pages 4,5; claim; fig. 1,2 * | 1,4 | |
| A | EP - A1 - 0 359 888 (PAPADOPOULOS) * Abstract; description; fig. 1-4 * | 1 | TECHNICAL FIELDS SEARCHED (Int. Cl.5) A 61 N 1/00 A 61 B 5/00 A 63 B 21/00 A 41 B 9/00 C 08 F 2/00 |
| A | DE - A1 - 3 628 216 (KOGAN) * Description; fig. 1,8 * | 1 | |
| A | GB - A - 823 946 (FLOWERS) * Claims; drawing * | 1 | |
| A | US - A - 4 964 173 (GORDON) * Fig. 10,11; column 6, lines 12-24 * | 3 | |
| A | DE - A1 - 3 510 031 | 15 | |

The present search report has been drawn up for all claims

| Place of search | Date of completion of the search | Examiner |
|---|---|---|
| VIENNA | 14-11-1991 | SCHÖNWÄLDER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

7

European Patent
Office

**EUROPEAN SEARCH REPORT**

Application Number

-2-

EP 91830039.3

| | **DOCUMENTS CONSIDERED TO BE RELEVANT** | | | |
|---|---|---|---|
| Category | Citation of document with indication, where appropriate, of relevant passages | Relevant to claim | CLASSIFICATION OF THE APPLICATION (Int. Cl.5) |
| | (BASF AG)<br>* Page 2; 4th,5th breaks * | | |

TECHNICAL FIELDS
SEARCHED (Int. Cl.5)

The present search report has been drawn up for all claims

| Place of search | Date of completion of the search | Examiner |
|---|---|---|
| VIENNA | 14-11-1991 | SCHÖNWÄLDER |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)